# EUROPEAN PATENT APPLICATION

(11) **EP 4 620 417 A1**
(43) Date of publication of application: **24.09.2025**
(21) Application number: 23891936.9
(22) Date of filing: 13.11.2023
(51) Int. Cl.: A61B 34/00, A61B 34/37, A61B 90/00

(54) **SURGICAL TOOL CONTROL SYSTEM COMPRISING OPERATING HANDLE**

(30) Priority: 14.11.2022 KR 20220151475
(71) Applicant: LN Robotics Inc., Seoul 05505 (KR)
(72) Inventor: WON, Jong Seok, Seoul 06226 (KR); CHOI, Ju Eun, Seoul 04906 (KR); PARK, Sang Eun, Seoul 05285 (KR)
(74) Representative: Lavoix
(86) International application number: PCT/KR2023/018134
(87) International publication number: WO 2024/106869

(57) **Abstract**

A surgical tool control system according to one embodiment comprises: a driving assembly enabling a mounted surgical tool to advance, retreat or rotate; and an operating handle for receiving, from a user, a command to be transmitted to the driving assembly, wherein the operating handle may generate respective surgical tool advancing or retreating signals when the operating handle advances or retreats along a first axis, and may generate respective clockwise or counterclockwise surgical tool rotation signals when the operating handle rotates clockwise or counterclockwise around the first axis.

## Description

### TECHNICAL FIELD

The following embodiment relates to a surgical tool control system including an operating handle.

### BACKGROUND ART

The existing percutaneous coronary intervention (PCI) procedure has the risk of continuous radiation exposure of operators, requires a great amount of time and cost to train skilled operators to the level where stable surgical procedures are possible, and has degrees of completion of the procedure with a large gap between operators/regions/hospitals, making it difficult to universally provide high-quality medical services. Interventional assistant robots have been introduced to compensate for the above disadvantages. For example, interventional assistant robots may be configured to move a surgical tool forward and backward or rotate the surgical tool when a user inputs a command. To increase the precision of a procedure using such interventional assistant robots, a structure that may improve the user's operational convenience will be required.

The above description is information the inventor(s) acquired during the course of conceiving the present disclosure, or already possessed at the time, and was not necessarily publicly known before the present application was filed.

### DISCLOSURE OF THE INVENTION

### TECHNICAL GOALS

The purpose of an embodiment is to provide a surgical tool control system with improved operational convenience for a user.

The purpose of an embodiment is to provide a surgical tool control system that allows a user to easily and precisely input a surgical tool control command.

### TECHNICAL SOLUTIONS

According to an embodiment, a surgical tool control system includes a driving assembly configured to move a surgical tool that is mounted forward and backward or rotate the surgical tool and an operating handle configured to receive a command to be transmitted from a user to the driving assembly, in which the operating handle is configured to generate a forward signal or a backward signal of the surgical tool, respectively, when the operating handle moves forward or backward along a first axis and generate a clockwise rotation signal or a counterclockwise rotation signal of the surgical tool, respectively, when the operating handle rotates in a clockwise direction or a counterclockwise direction around the first axis.

The surgical tool control system may further include a jog wheel that is coupled to the operating handle and rotatable around a second axis with respect to the operating handle, in which the jog wheel may be configured to generate a clockwise rotation signal or a counterclockwise rotation signal of the surgical tool, respectively, when the jog wheel rotates in a clockwise direction or a counterclockwise direction around the second axis.

The jog wheel may be configured to discretely rotate in a unit of a first angle around the second axis with respect to the operating handle, in which the jog wheel may be configured to generate a signal that rotates the surgical tool at a second angle when the jog wheel is rotated by the first angle.

The second angle may be set equal to the first angle or to be an angle obtained by multiplying a scale coefficient by the first angle.

The second angle may be 1 degree to 5 degrees.

The surgical tool control system may further include, in a state in which the driving assembly reaches a limit point in which the surgical tool is no longer rotated, a haptic actuator configured to provide haptic feedback to the jog wheel when an input that is greater than or equal to the limit point is input to the jog wheel.

The haptic feedback may be configured to provide rotational resistance in a direction that interferes with rotation of the jog wheel when the jog wheel is about to rotate further in a direction in which the limit point is reached.

The haptic feedback may be configured to reduce rotational resistance in a direction in which the jog wheel rotates when the jog wheel is about to rotate further in a direction in which the limit point is reached.

The haptic feedback may be configured to elastically return the jog wheel to an original position when the jog wheel rotates further in a direction in which the limit point is reached.

The surgical tool control system may further include a toggle switch configured to change a mode of the jog wheel, in which, depending on a state of the toggle switch, the jog wheel may be configured to generate a rotation signal of the surgical tool or a forward and backward signal of the surgical tool when the jog wheel rotates around the second axis.

The operating handle may be configured to change a rotation speed of the surgical tool in proportion to a rotation angle of the operating handle when the operating handle rotates around the first axis.

The operating handle may be configured to discretely change a rotation speed of the surgical tool according to a section to which a rotation angle of the operating handle belongs when the operating handle rotates around the first axis.

The operating handle may be configured to change a forward speed or a backward speed of the surgical tool according to a degree to which the operating handle moves forward or backward along the first axis.

The operating handle may be configured to discretely change a forward speed or a backward speed of the surgical tool according to a section to which a stroke in which the operating handle moves forward or backward along a center of the first axis belongs.

The surgical tool control system may further include a first jog wheel that is coupled to the operating handle and rotatable around a second axis with respect to the operating handle and a second jog wheel that is coupled to the operating handle and rotatable around a third axis with respect to the operating handle, in which one of the first jog wheel and the second jog wheel may be configured to generate a clockwise rotation signal or a counterclockwise rotation signal of the surgical tool and the other may be configured to generate a forward signal or a backward signal of the surgical tool.

The surgical tool control system may further include a first jog wheel that is coupled to the operating handle and rotatable around a second axis with respect to the operating handle and a second jog wheel that is coupled to the operating handle and rotatable around a third axis with respect to the operating handle, in which one of a first forward and backward operation of the surgical tool, a first rotation operation of the surgical tool, a second forward and backward operation of the surgical tool, and a second rotation operation of the surgical tool may be designated for a forward and backward movement of the operating handle, rotation of the operating handle, rotation of the first jog wheel, and rotation of the second jog wheel, respectively.

The driving assembly may include a first roller module and a second roller module, in which, in a state in which the surgical tool is gripped between the first roller module and the second roller module, the driving assembly may be configured to rotate the surgical tool by moving the surgical tool forward and backward by rotating the first roller module and the second roller module or moving at least one of the first roller module and the second roller module in a vertical direction.

The surgical tool control system may further include a jog wheel that is coupled to the operating handle and rotatable around a second axis with respect to the operating handle, in which the jog wheel may be configured to generate a forward signal or a backward signal of the surgical tool when the jog wheel rotates in a clockwise direction or a counterclockwise direction around the second axis.

The jog wheel may be configured to discretely rotate in a unit of a first angle around a second axis with respect to the operating handle, in which the jog wheel may be configured to generate a signal that moves the surgical tool forward and backward by a first pitch when the jog wheel is rotated by the first angle.

The first angle may be 1 degree to 5 degrees, and the first pitch may be 0.5 mm to 1.5 mm.

### EFFECTS OF THE INVENTION

According to an embodiment, a surgical tool control system may improve the operational convenience for a user.

According to an embodiment, a surgical tool control system may allow a user to easily and precisely input a surgical tool control command.

The effects of the surgical tool control system according to an embodiment are not limited to the above-mentioned effects, and other unmentioned effects may be clearly understood from the following description by one of ordinary skill in the art.

### BRIEF DESCRIPTION OF DRAWINGS

FIG. 1 is a schematic perspective view of a surgical tool control system according to an embodiment.
FIG. 2 is a perspective view of a driving assembly according to an embodiment.
FIG. 3 is a plan view of a driving assembly according to an embodiment.
FIG. 4 is a use state view of a driving assembly according to an embodiment.
FIG. 5 illustrates a process in which a roller module of a driving assembly moves a surgical tool forward and backward, according to an embodiment.
FIG. 6 illustrates a process in which a roller module of a driving assembly rotates a surgical tool, according to an embodiment.
FIG. 7 is a perspective view of an operating assembly according to an embodiment.
FIG. 8 is a perspective view of an operating assembly according to an embodiment.

### BEST MODE FOR CARRYING OUT THE INVENTION

Hereinafter, embodiments will be described in detail with reference to the accompanying drawings. However, various alterations and modifications may be made to the embodiments. Here, the embodiments are not construed as limited to the disclosure. Here, the embodiments are not construed as limited to the disclosure and should be understood to include all changes, equivalents, and replacements within the idea and the technical scope of the disclosure.

The terminology used herein is for the purpose of describing particular embodiments only and is not to be limiting of the embodiments. The singular forms "a", "an", and "the" are intended to include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms including technical and scientific terms used herein have the same meaning as commonly understood by one of ordinary skill in the art to which the embodiments belong. It will be further understood that terms, such as those defined in commonly-used dictionaries, should be interpreted as having a meaning that is consistent with their meaning in the context of the relevant art and will not be interpreted in an idealized or overly formal sense unless expressly so defined herein.

When describing the embodiments with reference to the accompanying drawings, like reference numerals refer to like constituent elements and a repeated description related thereto will be omitted. In the description of embodiments, detailed description of well-known related structures or functions will be omitted when it is deemed that such description will cause ambiguous interpretation of the present disclosure.

In addition, terms such as first, second, A, B, (a), (b), and the like may be used to describe components of the embodiments. These terms are used only for the purpose of discriminating one component from another component, and the nature, the sequences, or the orders of the components are not limited by the terms. When one component is described as being "connected", "coupled", or "attached" to another component, it should be understood that one component may be connected or attached directly to another component, and an intervening component may also be "connected", "coupled", or "attached" to the components.

The same name may be used to describe an element included in the embodiments described above and an element having a common function. Unless otherwise mentioned, the descriptions on the embodiments may be applicable to the following embodiments and thus, duplicated descriptions will be omitted for conciseness.

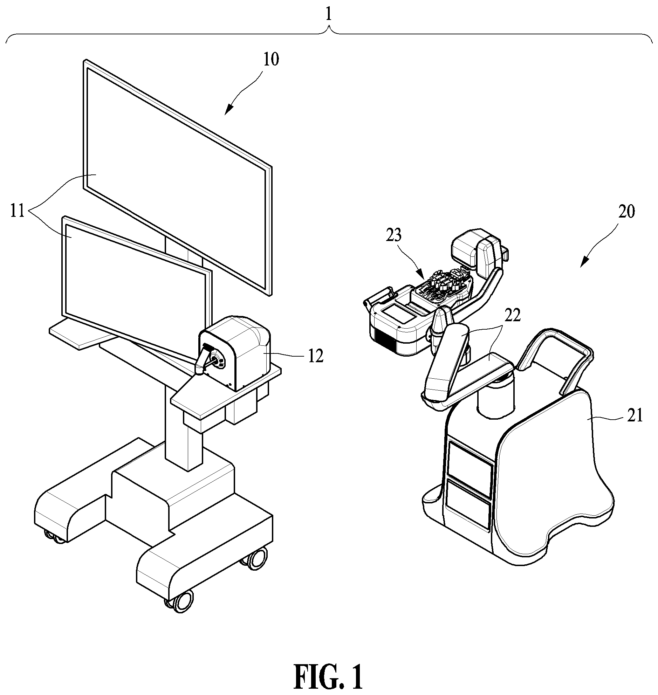

FIG. 1 is a schematic perspective view of a surgical tool control system according to an embodiment.

Referring to FIG. 1, a surgical tool control system 1 according to an embodiment may receive an operation command from a user and drive a surgical tool according to the input command. For example, the surgical tool control system 1 may be used for a percutaneous coronary intervention (PCI) procedure. However, this is an example, and the use of the surgical tool control system 1 is not limited thereto.

In an embodiment, the surgical tool control system 1 may include a master part 10 and a slave part 20. The master part 10 may receive commands from a user and display information to the user. The slave part 20 may receive commands from the master part 10 and drive the surgical tool according to the received commands. For example, the slave part 20 may be positioned in a surgical space with X-ray equipment, and the master part 10 may be positioned in a shielded space that is separated from the surgical space. The user may input commands through the master part 10 in the shielded space so that the user may not be exposed to radiation from X-rays.

In an embodiment, the master part 10 may include a display 11 and an operating assembly 12. The display 11 may provide visual information to the user. For example, the display 11 may display information about the slave part 20 or display an X-ray image. The display 11 may receive an input from the user through a touch panel. The operating assembly 12 may receive commands from the user to operate the surgical tool. The operating assembly 12 may transmit the commands received from the user to a driving assembly 23.

In an embodiment, the slave part 20 may include a slave base 21, an arm 22, and the driving assembly 23. The slave base 21 may provide a base on which the slave part 20 is installed. The driving assembly 23 may be connected to the slave base 21 through at least one arm 22. For example, the at least one arm 22 may connect the driving assembly 23 to the slave base 21 through a link structure. The position of the driving assembly 23 with respect to the slave base 21 may be adjusted by the at least one arm 22. At least one surgical tool may be mounted on the driving assembly 23. The driving assembly 23 may drive the at least one mounted surgical tool. For example, the driving assembly 23 may move the surgical tool forward and backward or rotate the surgical tool. The driving assembly 23 may receive commands from the master part 10 and drive the at least one surgical tool according to the received commands.

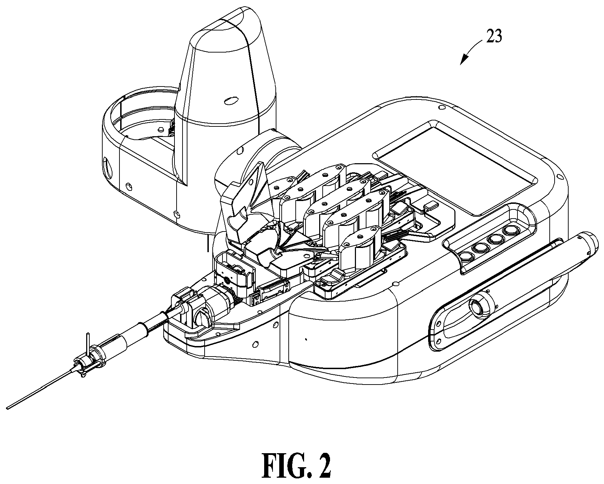

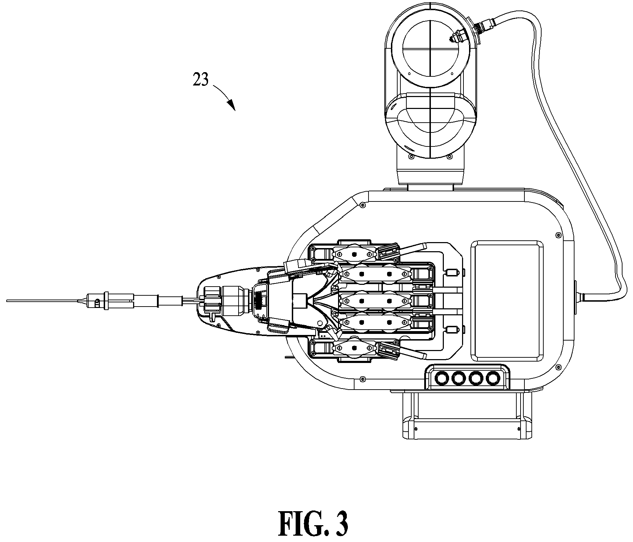

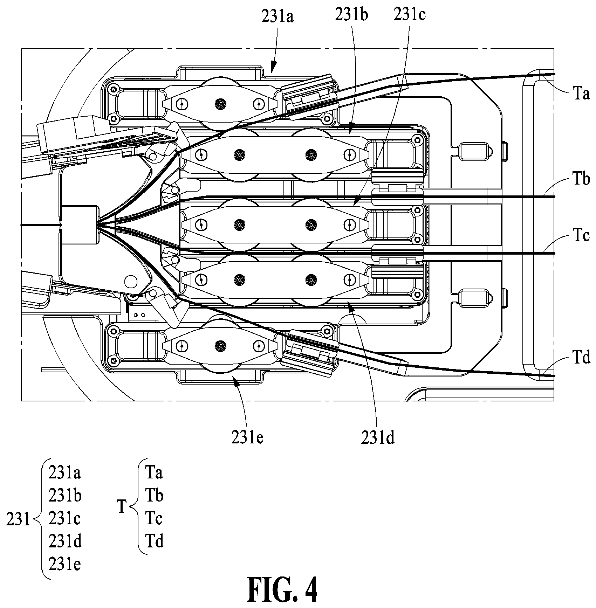

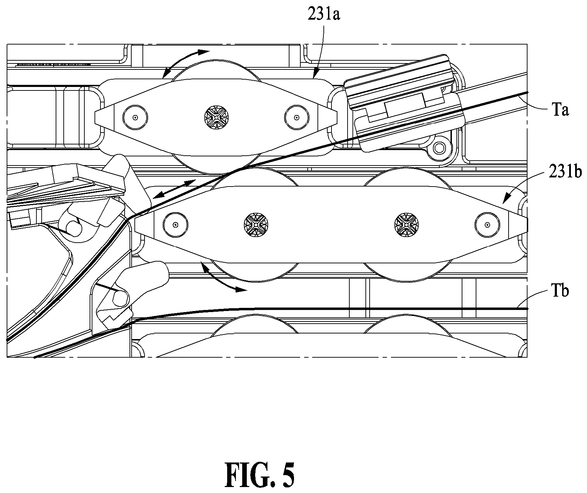

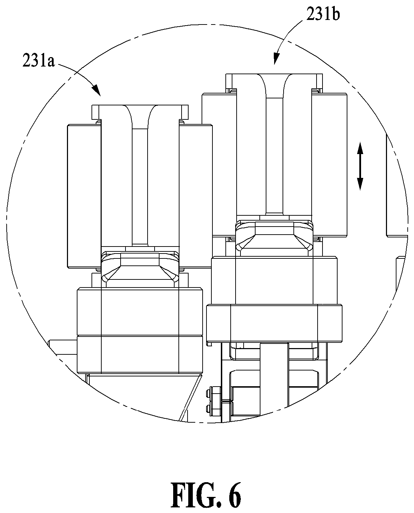

FIG. 2 is a perspective view of a driving assembly according to an embodiment. FIG. 3 is a plan view of a driving assembly according to an embodiment. FIG. 4 is a use state view of a driving assembly according to an embodiment. FIG. 5 illustrates a process in which a roller module of a driving assembly moves a surgical tool forward and backward, according to an embodiment. FIG. 6 illustrates a process in which a roller module of a driving assembly rotates a surgical tool, according to an embodiment.

Referring to FIGS. 2 to 6, the driving assembly 23 may independently control a plurality of surgical tools T. Here, the surgical tools T may refer to surgical tools having a longitudinal direction. For example, the surgical tools T may include various surgical tools, such as a guide wire and a balloon catheter having a longitudinal direction. However, this is an example, and the types of surgical tools T are not limited thereto.

In an embodiment, the driving assembly 23 may include a plurality of roller modules 231. At least one pair of the roller modules 231 may be provided. For example, five roller modules 231 may be provided, as shown in the drawing. However, this is an example, and the number of roller modules 231 is not limited thereto. The plurality of roller modules 231 may be arranged in parallel with each other.

In an embodiment, the surgical tools T may be gripped between two adjacent roller modules 231. For this, at least one roller module 231 may be horizontally movable with respect to another roller module 231. For example, as shown in FIG. 4, a second roller module 232b may be horizontally movable with respect to a first roller module 231a so that a first surgical tool Ta is gripped between the first roller module 231a and the second roller module 231b. In this case, a second surgical tool Tb positioned between the second roller module 231b and a third roller module 231c may be released. In addition, on the contrary, the second roller module 231b may be horizontally movable with respect to the third roller module 231c so that the second surgical tool Tb is gripped between the second roller module 231b and the third roller module 231c. In this case, the first surgical tool Ta positioned between the first roller module 231a and the second roller module 231b may be released.

Similarly, a fourth roller module 231d may be horizontally movable with respect to the third roller module 231c so that a third surgical tool Tc is gripped between the third roller module 231c and the fourth roller module 231d. In this case, a fourth surgical tool Td positioned between the fourth roller module 231d and a fifth roller module 231e may be released. In addition, on the contrary, the fourth roller module 231d may be horizontally movable with respect to the fifth roller module 231e so that the fourth surgical tool Td is gripped between the fourth roller module 231d and the fifth roller module 231e. In this case, the third surgical tool Tc positioned between the third roller module 231c and the fourth roller module 231d may be released.

In an embodiment, the driving assembly 23 may implement a forward and backward movement and rotation of the surgical tools T through the rotation and vertical movement of the roller module 231. Specifically, as shown in FIG. 5, in a state in which the first roller module 231a and the second roller module 231b are positioned adjacent to each other to grip the first surgical tool Ta therebetween, the first roller module 231a and the second roller module 231b rotate in one direction or the other direction, thereby moving the first surgical tool Ta gripped therebetween forward or backward along the longitudinal direction. In addition, as shown in FIG. 6, in a state in which the first roller module 231a and the second roller module 231b are positioned adjacent to each other to grip the first surgical tool Ta therebetween, at least one of the roller modules 231a and 231b may move in the vertical direction, thereby rotating the first surgical tool Ta gripped therebetween.

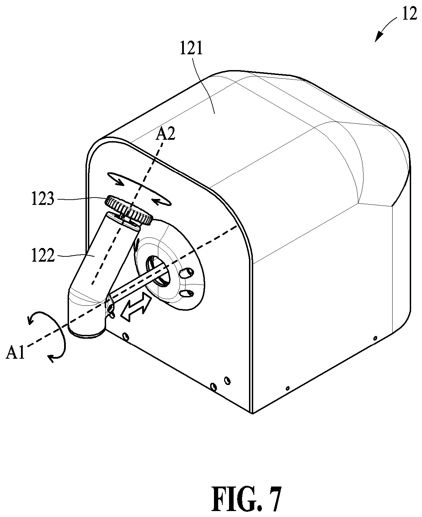

FIG. 7 is a perspective view of an operating assembly according to an embodiment.

Referring to FIGS. 1 and 7, the operating assembly 12 according to an embodiment may receive commands from a user to drive a surgical tool. The operating assembly 12 may transmit the received commands to the driving assembly 23. The operating assembly 12 may include a base housing 121, an operating handle 122, and a jog wheel 123.

In an embodiment, the base housing 121 may form at least a portion of the exterior of the operating assembly 12. The base housing 121 may be fixedly installed on the master part 10. The base housing 121 may provide a space in which various components may be disposed therein. For example, components, such as a shaft, a gear, and/or a motor, may be disposed inside the base housing 121.

In an embodiment, the operating handle 122 may receive, from the user, commands to be transmitted to the driving assembly 23. The operating handle 122 may be formed in a shape that is easy for the user to grip with one hand. Furthermore, the position, shape, and/or size of the operating handle 122 illustrated in the drawing are examples and are not limited to those illustrated in the drawing. The operating handle 122 may be connected to the base housing 121 to be able to move forward and backward and/or to be rotatable.

In an embodiment, the operating handle 122 may move forward or backward with respect to the base housing 121 along a first axis A1. However, the direction of the first axis A1 shown in the drawing is an example, and the direction of the first axis A1 is not limited thereto. The operating handle 122 may generate a forward signal or a backward signal of the surgical tool, respectively, when the operating handle 122 moves forward or backward along the first axis A1. The forward signal or the backward signal generated from the operating handle 122 may be transmitted to the driving assembly 23, and the roller modules 231 may rotate so that the surgical tool gripped by roller modules (e.g., the roller modules 231 of FIG. 4) moves forward or backward. According to this configuration, when the user moves the operating handle 122 forward along the first axis A1, the surgical tool may move forward, and when the user moves the operating handle 122 backward along the first axis A1, the surgical tool may move backward. According to the degree to which the operating handle 122 moves forward or backward along the first axis A1, the operating handle 122 may change a forward speed or a backward speed of the surgical tool. For example, the operating handle 122 may change the forward speed or the backward speed of the surgical tool in proportion to a stroke in which the operating handle 122 moves forward or backward along the first axis A1. Alternatively, the operating handle 122 may discretely change the forward speed or the backward speed of the surgical tool according to a section to which the stroke in which the operating handle 122 moves forward or backward along the first axis A1 belongs. For example, the forward speed or the backward speed of the surgical tool may be discretely changed by 0.5 times, 1 time, or 2 times according to a section to which the stroke in which the operating handle 122 moves forward or backward along the first axis A1 belongs. A method in which the forward speed or the backward speed of the surgical tool is changed according to the degree to which the operating handle 122 moves forward or backward along the first axis A1 may be changed according to a user's settings.

In an embodiment, the operating handle 122 may be rotatable in a clockwise direction or a counterclockwise direction with respect to the base housing 121 around the first axis A1. The operating handle 122 may generate a clockwise rotation signal or a counterclockwise rotation signal of the surgical tool, respectively, when the operating handle 122 rotates in a clockwise direction or a counterclockwise direction around the first axis A1. The clockwise rotation signal or the counterclockwise rotation signal generated from the operating handle 122 may be transmitted to the driving assembly 23, and at least one of the roller modules 231 may move in a vertical direction so that the surgical tool gripped by the roller modules (e.g., the roller modules 231 of FIG. 4) rotates in a clockwise direction or a counterclockwise direction. According to this configuration, when the user rotates the operating handle 122 in a clockwise direction around the first axis A1, the surgical tool may rotate in a clockwise direction, and when the user rotates the operating handle 122 in a counterclockwise direction around the first axis A1, the surgical tool may rotate in a counterclockwise direction. According to the degree to which the operating handle 122 rotates around the first axis A1, the operating handle 122 may change a rotation speed of the surgical tool. For example, the operating handle 122 may change the rotation speed of the surgical tool in proportion to a rotation angle of the operating handle 122. Alternatively, the operating handle 122 may discretely change the rotation speed of the surgical tool according to a section to which the rotation angle of the operating handle 122 belongs. For example, when the rotation angle of the operating handle 122 belongs to a first section, the rotation speed of the surgical tool may be set to a first speed, and when the rotation angle of the operating handle 122 belongs to a second section, the rotation speed of the surgical tool may be set to a second speed. A method in which the rotation speed of the surgical tool is changed according to the degree to which the operating handle 122 rotates may be changed according to a user's settings.

In an embodiment, in a state in which the operating handle 122 is moved forward, backward, or rotated, the surgical tool may be consecutively moved forward, backward, or rotated. When the force applied to the operating handle 122 disappears, the operating handle 122 may be returned to an original position. For example, when the user moves the operating handle 122 forward and backward or rotates the operating handle 122 and then releases the force, the operating handle 122 may return to the original position. The driving of the surgical tool may be stopped when the operating handle 122 is returned to the original position.

In an embodiment, the jog wheel 123 may be coupled to the operating handle 122. The jog wheel 123 may be rotatable in a clockwise direction or a counterclockwise direction with respect to the operating handle 122 around a second axis A2. The finger (e.g., the thumb or index finger) of the user may be positioned on the jog wheel 123 when the user grips the operating handle 122 with their hand. Furthermore, the position, shape, and/or size of the jog wheel 123 illustrated in the drawing are examples and are not limited to those illustrated in the drawing. In addition, the direction of the second axis A2 shown in the drawing is an example, and the direction of the second axis A2 is not limited thereto.

In an embodiment, the jog wheel 123 may generate a clockwise rotation signal or a counterclockwise rotation signal of the surgical tool, respectively, when the jog wheel 123 rotates in a clockwise direction or a counterclockwise direction around the second axis A2. The clockwise rotation signal or the counterclockwise rotation signal generated from the jog wheel 123 may be transmitted to the driving assembly 23, and at least one of the roller modules 231 may move in a vertical direction so that the surgical tool gripped by the roller modules (e.g., the roller modules 231 of FIG. 4) rotates in a clockwise direction or a counterclockwise direction. According to this configuration, when the user rotates the jog wheel 123 in a clockwise direction around the second axis A2, the surgical tool may rotate in a clockwise direction, and when the user rotates the jog wheel 123 in a counterclockwise direction around the second axis A2, the surgical tool may rotate in a counterclockwise direction.

In an embodiment, the jog wheel 123 may generate a signal to drive the surgical tool more precisely than the operating handle 122. The jog wheel 123 may discretely rotate in a unit of a first angle with respect to the operating handle 122 around the second axis A2. Since the jog wheel 123 is configured to discretely rotate in the unit of the first angle, the user may rotate the jog wheel 123 by a unit of one space (i.e., the unit of the first angle). For example, the first angle may be 5 degrees. With this configuration, the jog wheel 123 may have a total of 72 rotation sections. However, this is an example, and the first angle is not limited thereto.

In an embodiment, the jog wheel 123 may generate a signal to rotate the surgical tool at a second angle when the jog wheel 123 is rotated by the first angle. That is, each time the user rotates the jog wheel 123 by one space (i.e., the first angle), the surgical tool may be rotated by the second angle. The second angle may be set equal to the first angle. For example, when the first angle is 5 degrees, the second angle may also be set to 5 degrees, the same as the first angle. Alternatively, the second angle may be set as an angle obtained by multiplying a scale coefficient by the first angle. For example, the scale coefficient may be set to a number less than 1. For example, when the first angle is 5 degrees, the second angle may be set to 1 degree. Desirably, the second angle may be set in a range of 1 to 5 degrees. However, this is an example, and the second angle is not limited thereto. The second angle may be changed in various ways according to a user's settings.

In an embodiment, the operating assembly 12 may further include a haptic actuator (not shown). The haptic actuator may provide haptic feedback to the jog wheel 123. For example, referring to FIG. 6, since there is a physical limit to the degree to which the roller modules 231a and 231b may move in the vertical direction, when the surgical tool continuously rotates in one direction, the driving assembly 23 may reach a limit point in which the surgical tool may no longer be rotated. As described above, in a state in which the driving assembly 23 reaches the limit point in which the surgical tool may no longer be rotated, the haptic actuator may provide the haptic feedback to the jog wheel 123 when an input that is greater than or equal to the limit point is input to the jog wheel 123.

In an embodiment, the haptic feedback may provide rotational resistance in a direction that interferes with the rotation of the jog wheel 123 when the jog wheel 123 is about to rotate further in a direction in which the limit point is reached. For example, when the jog wheel 123 is about to rotate further in a direction in which the limit point is reached, the haptic actuator may rotate the jog wheel 123 as if bouncing in the opposite direction or generate a motor torque in the opposite direction to cause the user to feel rotational resistance.

In an embodiment, the haptic feedback may reduce rotational resistance in a direction in which the jog wheel 123 rotates when the jog wheel 123 is about to rotate further in a direction in which the limit point is reached. For example, when the jog wheel 123 is about to rotate further in a direction in which the limit point is reached, the haptic actuator may reduce the rotational resistance in a direction in which the jog wheel 123 rotates to cause the user to feel as if the jog wheel 123 is spinning with no traction.

In an embodiment, the haptic feedback may elastically return the jog wheel 123 to the original position (e.g., a limit point position) when the jog wheel 123 rotates further in a direction in which the limit point is reached. For example, in a state in which the driving assembly 23 reaches the limit point in which the surgical tool may no longer be rotated, when an input that is greater than or equal to the limit point is input to the jog wheel 123, the jog wheel 123 may rotate partly or completely beyond the limit point in response to the input but may be elastically returned to the original position (e.g., a limit point position) by an actuator. For example, the actuator may elastically return the jog wheel 123 to the original position (e.g., a limit point position) so that the reciprocating width gradually attenuates as the jog wheel 123 reciprocates back and forth from the original position (e.g., a limit point position).

According to the above-described configuration, the user may easily recognize that a driving limit point is reached by the haptic feedback provided by the haptic actuator to the jog wheel 123. Furthermore, the haptic feedback is described for the jog wheel 123, but the haptic feedback may be applied to the operating handle 122 in substantially the same manner. For example, in a state in which the driving limit point is reached, when an input that is greater than or equal to the limit point is input through the operating handle 122, the haptic actuator may provide the haptic feedback to the operating handle 122.

In an embodiment, the master part 10 may further include a toggle switch (not shown). The toggle switch may be implemented as a physical switch in the operating assembly 12. Alternatively, the toggle switch may be implemented as a virtual switch that appears on the display 11. The toggle switch may change a mode of the jog wheel 123. For example, the jog wheel 123 may have a rotation mode or a forward and backward mode depending on a state of the toggle switch.

In an embodiment, when the jog wheel 123 is in a rotation mode, a rotation signal of the surgical tool may be generated when the jog wheel 123 rotates around the second axis A2. The rotation mode of the jog wheel 123 is the same as that described above, so the above description is applied mutatis mutandis.

In an embodiment, when the jog wheel 123 is in a forward and backward mode, a forward and backward signal of the surgical tool may be generated when the jog wheel 123 rotates around the second axis A2. For example, the jog wheel 123 may generate a signal to move the surgical tool forward or backward by a first pitch, respectively, when the jog wheel 123 rotates in a clockwise direction or a counterclockwise direction by the first angle. That is, when the jog wheel 123 is in the forward and backward mode, each time the user rotates the jog wheel 123 by one space (i.e., the first angle), the surgical tool may be moved forward and backward by the first pitch. The first pitch, which is a distance in which the surgical tool moves forward and backward per one space rotation of the jog wheel 123, may be changed in various ways according to a user's settings. For example, the first angle may be between 1 degree and 5 degrees. For example, the first pitch may be 0.5 mm to 1.5 mm. For example, the first angle may be 5 degrees, and the first pitch may be 1 mm.

In an embodiment, the operating assembly 12 may include a trackball (not shown). For example, the trackball may be installed on the base housing 121 or the operating handle 122. The trackball may be rotatable in any direction. For example, when the trackball rotates in a left and right direction, the trackball may generate a rotation signal of the surgical tool. For example, when the trackball rotates in a vertical direction, the trackball may generate a forward and backward signal of the surgical tool. When the trackball rotates in a complex direction, the trackball may generate a rotation signal and a forward and backward signal of the surgical tool, respectively, according to the left and right direction component and the vertical direction component.

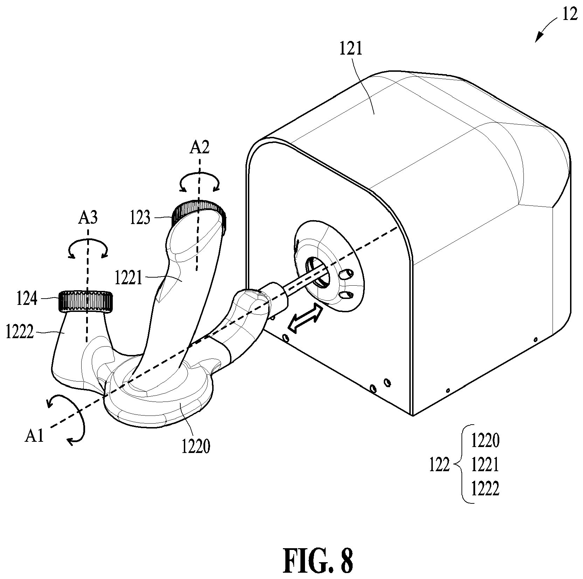

FIG. 8 is a perspective view of an operating assembly according to an embodiment.

Referring to FIG. 8, the operating assembly 12 according to an embodiment may receive commands from a user to drive a surgical tool. The operating assembly 12 may transmit the received commands to the driving assembly 23. The operating assembly 12 may include the base housing 121, the operating handle 122, a first jog wheel 123, and a second jog wheel 124.

Hereinafter, to avoid excessive repetition, a detailed description of the same configuration as the operating assembly 12 provided with reference to FIG. 7 is omitted, and the description of the operating assembly 12 provided with reference to FIG. 7 may apply to the description of the operating assembly 12 provided with reference to FIG. 8 within a range that does not contradict each other.

Referring to FIG. 8, in an embodiment, the operating handle 122 may receive, from the user, commands to be transmitted to the driving assembly 23. For example, the operating handle 122 may include a handle base 1220, a first handle 1221, and a second handle 1222. The handle base 1220 may refer to a base portion of the operating handle 122. The first handle 1221 may extend upward from the handle base 1220. The first handle 1221 may be formed in a shape that is easy for the user to grip with one hand. For example, the first handle 1221 may extend from the handle base 1220 to incline forward. The second handle 1222 may extend from one side of the handle base 1220. For example, the second handle 1222 may be formed to have a smaller size (e.g., a height) than the first handle 1221. For example, the user may operate the operating handle 122 by gripping the first handle 1221 with one hand. For example, the user may move the operating handle 122 forward and/or backward with respect to the base housing 121 along the first axis A1 or rotate the operating handle 122 around the first axis A1. Furthermore, the position, shape, and/or size of the operating handle 122 illustrated in the drawing are examples and are not limited to those illustrated in the drawing. For example, the second handle 1222 may be spaced apart from the handle base 1220 and configured as a separate handle. For example, the first handle 1221 and the second handle 1222 may each be configured as a separate handle such that the user may grip the first handle 1221 with one hand and the second handle 1222 with the other hand.

In an embodiment, the first jog wheel 123 may be coupled to the first handle 1221. The first jog wheel 123 may be rotatable in a clockwise direction or a counterclockwise direction with respect to the first handle 1221 around the second axis A2. When the user grips the first handle 1221 with their hand, the finger (e.g., the thumb or index finger) of the user may be positioned on the first jog wheel 123. The configuration of the first jog wheel 123 is substantially the same as the jog wheel 123 described with reference to FIG. 7, so the description of the jog wheel 123 provided with reference to FIG. 7 is applied mutatis mutandis. For example, the first jog wheel 123 may generate a forward and backward signal (or a rotation signal) of the surgical tool when the first jog wheel 123 rotates around the second axis A2.

In an embodiment, the second jog wheel 124 may be coupled to the second handle 1222. The second jog wheel 124 may rotate in a clockwise direction or a counterclockwise direction with respect to the second handle 1222 around a third axis A3. For example, the user may control the operating handle 122 and/or the first jog wheel 123 by gripping the first handle 1221 with one hand (e.g., the right hand) and control the second jog wheel 124 positioned on the second handle 1222 with the other hand (e.g., the left hand). However, this is an example, and the gripping method of the user is not limited thereto. The second jog wheel 124 may perform substantially the same function as the jog wheel 123 described with reference to FIG. 7. Accordingly, the description of the jog wheel 123 provided with reference to FIG. 7 may apply to the description of the function of the second jog wheel 124. For example, the second jog wheel 124 may generate a rotation signal (or a forward and backward signal) of the surgical tool when the second jog wheel 124 rotates around the third axis A3.

In an embodiment, the first jog wheel 123 and the second jog wheel 124 may be configured to generate different driving signals. For example, the first jog wheel 123 may generate a driving signal to move the surgical tool forward, and the second jog wheel 124 may generate a driving signal to rotate the surgical tool. For example, the first jog wheel 123 and the second jog wheel 124 may be configured to control the surgical tool more precisely than the control of the surgical tool by the forward and backward movement and rotation of the operating handle 122. For example, the first jog wheel 123 may generate a signal to move the surgical tool forward and backward to a smaller degree than the forward and backward degree of the surgical tool according to the forward and backward movement of the operating handle 122. For example, the second jog wheel 124 may generate a signal to rotate the surgical tool to a smaller degree than the rotation degree of the surgical tool according to the rotation of the operating handle 122. However, this is an example, and conversely, the first jog wheel 123 may generate a driving signal to rotate the surgical tool, and the second jog wheel 124 may generate a driving signal to move the surgical tool forward. For example, one of the first jog wheel 123 and the second jog wheel 124 may generate a clockwise rotation signal or a counterclockwise rotation signal of the surgical tool, and the other may generate a forward signal or a backward signal of the surgical tool.

In an embodiment, the user may designate any operation control of the surgical tool for the forward and backward movement of the operating handle 122, the rotation of the operating handle 122, the rotation of the first jog wheel 123, and the rotation of the second jog wheel 124. For example, one of a first forward and backward operation of the surgical tool, a first rotation operation of the surgical tool, a second forward and backward operation of the surgical tool, and a second rotation operation of the surgical tool may be designated for the forward and backward movement of the operating handle 122, the rotation of the operating handle 122, the rotation of the first jog wheel 123, and the rotation of the second jog wheel 124, respectively. For example, the first forward and backward operation may be an operation that moves the surgical tool forward and backward to a relatively large degree compared to the second forward and backward operation, and the second forward and backward operation may be an operation that moves the surgical tool forward and backward to a relatively small degree compared to the first forward and backward operation. For example, the first rotation operation may be an operation that rotates the surgical tool to a relatively large degree compared to the second rotation operation, and the second rotation operation may be an operation that rotates the surgical tool to a relatively small degree compared to the first rotation operation. For example, the user may set the operating assembly 12 to move the surgical tool forward and backward to a relatively large degree by moving the operating handle 122 forward and backward, rotate the surgical tool to a relatively large degree by rotating the operating handle 122, move the surgical tool forward and backward to a relatively small degree by rotating the first jog wheel 123, and rotate the surgical tool to a relatively small degree by rotating the second jog wheel 124. However, this is an example, and the operation control of the surgical tool designated in each operating method is not limited thereto. For example, the user may not designate the operation control of the surgical tool in some operating methods if needed. For example, the user may control the surgical tool only with the first jog wheel 123 and the second jog wheel 124 without designating the operation control of the surgical tool for the forward and backward movement of the operating handle 122 and the rotation of the operating handle 122.

While the embodiments are described with reference to drawings, it will be apparent to one of ordinary skill in the art that various alterations and modifications in form and details may be made in these embodiments without departing from the spirit and scope of the claims and their equivalents. For example, suitable results may be achieved if the described techniques are performed in a different order, and/or if components in a described system, architecture, device, or circuit are combined in a different manner, and/or rearranged or supplemented by other components or their equivalents.

Therefore, other implementations, other embodiments, and equivalents to the claims are also within the scope of the following claims.

## Claims

1. A surgical tool control system comprising:
a driving assembly configured to move a surgical tool that is mounted forward and backward or rotate the surgical tool; and
an operating handle configured to receive a command to be transmitted from a user to the driving assembly,
wherein the operating handle is configured to:
generate a forward signal or a backward signal of the surgical tool, respectively, when the operating handle moves forward or backward along a first axis; and
generate a clockwise rotation signal or a counterclockwise rotation signal of the surgical tool, respectively, when the operating handle rotates in a clockwise direction or a counterclockwise direction around the first axis.

2. The surgical tool control system of claim 1, further comprising:
a jog wheel that is coupled to the operating handle and rotatable around a second axis with respect to the operating handle,
wherein the jog wheel is configured to generate a clockwise rotation signal or a counterclockwise rotation signal of the surgical tool, respectively, when the jog wheel rotates in a clockwise direction or a counterclockwise direction around the second axis.

3. The surgical tool control system of claim 2, wherein the jog wheel is configured to discretely rotate in a unit of a first angle around the second axis with respect to the operating handle, wherein the jog wheel is configured to generate a signal that rotates the surgical tool at a second angle when the jog wheel is rotated by the first angle.

4. The surgical tool control system of claim 3, wherein the second angle is set equal to the first angle or to be an angle obtained by multiplying a scale coefficient by the first angle.

5. The surgical tool control system of claim 4, wherein the second angle is 1 degree to 5 degrees.

6. The surgical tool control system of claim 2, further comprising: in a state in which the driving assembly reaches a limit point in which the surgical tool is no longer rotated, a haptic actuator configured to provide haptic feedback to the jog wheel when an input that is greater than or equal to the limit point is input to the jog wheel.

7. The surgical tool control system of claim 6, wherein the haptic feedback is configured to provide rotational resistance in a direction that interferes with rotation of the jog wheel when the jog wheel is about to rotate further in a direction in which the limit point is reached.

8. The surgical tool control system of claim 6, wherein the haptic feedback is configured to reduce rotational resistance in a direction in which the jog wheel rotates when the jog wheel is about to rotate further in a direction in which the limit point is reached.

9. The surgical tool control system of claim 6, wherein the haptic feedback is configured to elastically return the jog wheel to an original position when the jog wheel rotates further in a direction in which the limit point is reached.

10. The surgical tool control system of claim 2, further comprising:
a toggle switch configured to change a mode of the jog wheel, wherein, depending on a state of the toggle switch, the jog wheel is configured to generate a rotation signal of the surgical tool or a forward and backward signal of the surgical tool when the jog wheel rotates around the second axis.

11. The surgical tool control system of claim 1, wherein the operating handle is configured to change a rotation speed of the surgical tool in proportion to a rotation angle of the operating handle when the operating handle rotates around the first axis.

12. The surgical tool control system of claim 1, wherein the operating handle is configured to discretely change a rotation speed of the surgical tool according to a section to which a rotation angle of the operating handle belongs when the operating handle rotates around the first axis.

13. The surgical tool control system of claim 1, wherein the operating handle is configured to change a forward speed or a backward speed of the surgical tool according to a degree to which the operating handle moves forward or backward along the first axis.

14. The surgical tool control system of claim 13, wherein the operating handle is configured to discretely change a forward speed or a backward speed of the surgical tool according to a section to which a stroke in which the operating handle moves forward or backward along a center of the first axis belongs.

15. The surgical tool control system of claim 1, further comprising:
a first jog wheel that is coupled to the operating handle and rotatable around a second axis with respect to the operating handle; and
a second jog wheel that is coupled to the operating handle and rotatable around a third axis with respect to the operating handle,
wherein one of the first jog wheel and the second jog wheel is configured to generate a clockwise rotation signal or a counterclockwise rotation signal of the surgical tool and the other is configured to generate a forward signal or a backward signal of the surgical tool.

16. The surgical tool control system of claim 1, further comprising:
a first jog wheel that is coupled to the operating handle and rotatable around a second axis with respect to the operating handle; and
a second jog wheel that is coupled to the operating handle and rotatable around a third axis with respect to the operating handle,
wherein one of a first forward and backward operation of the surgical tool, a first rotation operation of the surgical tool, a second forward and
backward operation of the surgical tool, and a second rotation operation of the surgical tool is designated for a forward and backward movement of the operating handle, rotation of the operating handle, rotation of the first jog wheel, and rotation of the second jog wheel, respectively.

17. The surgical tool control system of claim 1, wherein the driving assembly comprises a first roller module and a second roller module, wherein, in a state in which the surgical tool is gripped between the first roller module and the second roller module, the driving assembly is configured to rotate the surgical tool by moving the surgical tool forward and backward by rotating the first roller module and the second roller module or by moving at least one of the first roller module and the second roller module in a vertical direction.

18. The surgical tool control system of claim 1, further comprising:
a jog wheel that is coupled to the operating handle and rotatable around a second axis with respect to the operating handle,
wherein the jog wheel is configured to generate a forward signal or a backward signal of the surgical tool when the jog wheel rotates in a clockwise direction or a counterclockwise direction around the second axis.

19. The surgical tool control system of claim 18, the jog wheel is configured to discretely rotate in a unit of a first angle around a second axis with respect to the operating handle, wherein the jog wheel is configured to generate a signal that moves the surgical tool forward and backward by a first pitch when the jog wheel is rotated by the first angle.

20. The surgical tool control system of claim 19, wherein the first angle is 1 degree to 5 degrees, and the first pitch is 0.5 mm to 1.5 mm.
